Europäisches Patentamt

European Patent Office

Office européen des brevets

(11) Publication number: **0 373 927**
**A2**

## EUROPEAN PATENT APPLICATION

(21) Application number: **89313067.4**

(22) Date of filing: **14.12.89**

(51) Int. Cl.5: **A61B 17/22**

(30) Priority: **14.12.88 GB 8829182**

(43) Date of publication of application:
**20.06.90 Bulletin 90/25**

(84) Designated Contracting States:
**DE FR GB NL**

(71) Applicant: **MEDTRONIC, INC.**
**7000 Central Avenue N.E.**
**Minneapolis Minnesota 55432(US)**

(72) Inventor: **Shiu, Man Fai**
**39 Dyott Road**
**Moseley Birmingham B13 9Q Z(GB)**

(74) Representative: **Pearce, Anthony Richmond et al**
**MARKS & CLERK Alpha Tower Suffolk Street Queensway Birmingham B1 1TT(GB)**

(54) **Medical device.**

(57) A medical device for clearing obstructions in body vessels comprises a generally cylindrical housing (10) having a rotary screw (30) mounted therein. The screw (30) co-operates with an inner leading edge (17) of the housing (10) to provide a scissor-like cutting action. A tip region of the screw (30) projects forwardly of the housing (10) and is tapered for entering material to be removed. The tip region is of relatively blunt bullet-shape.

Fig.1

## MEDICAL DEVICE

This invention relates to a surgical instrument or medical device for removing material from a body vessel, particularly for clearing obstructions in ducts, eg arteries.

A number of techniques are known for clearing obstructions in coronary arteries. These include by-pass surgery, the use of a "balloon" to expand the arterial wall in the region of the obstruction, the use of a cutter passed along the artery to remove the obstruction, and the use of a drill which is also passed along the artery to pulverise the obstruction (see for example, US-A-4842579 and US-A-4784636). By-pass surgery is a major operation and is expensive. The use of surgical instruments in the above-described alternative methods involves the risk that the instrument which is passed along the artery will puncture the arterial wall during use or during passage along the artery to the location at which the obstruction is to be cleared. Additionally, the use of a drill which pulverises the obstruction involves the risk of the pulverised material lodging in another part of the body and causing damage there.

US-A-4653496 discloses a surgical instrument for removing vascular stenotic or occlusive lesions, wherein a number of different constructions are provided to be selected according to the character of the lesion to be removed. In one construction, a generally cylindrical housing of the instrument has a sharpened inner leading edge. A cutting helix formed of a wire of isosceles trapezoidal cross-section which is helically wound to provide an open helix having flat and smooth outer surfaces, is rotatably mounted in the housing. The helix has a tip region of constant cross-section which projects forwardly out of the housing to terminate at a forward end at which a more or less sharpened tip of the wire is provided. A scissor-like cutting action is provided between the outer surface of the helix and the inner leading edge of the housing to supplement the cutting action produced by the design of the cutting helix itself. This design of helix requires a relatively sharp tip to the wire in order to permit penetration into the material to be excised. Thus, there is a risk of vascular wall penetration with this design of instrument.

US-A-4669469 and US-A-4781186 disclose atherectomy devices in which a rotary and axially movable cutter is mounted wholly within a housing having a cut out in a side wall thereof, and an inflatable balloon is disposed on a side of the housing opposite to the cut-out. In use, the device is passed along a vessel to the site of an atheroma, the balloon is inflated to cause atheroma material to enter the cut-out, and then the cutter rotated and axially slid within the housing to excise the material in the housing. This tends to be a somewhat laborious task when extensive atheromas are encountered.

US-A-4765332 discloses an atherectomy apparatus wherein a rearwardly facing cutting cylinder spaced outwardly of a rearwardly opening chamber is provided in a guiding catheter which removes material by means of a planing action when the catheter is pulled backward.

It is an object of the present invention to provide a novel medical device for clearing obstructions in ducts in which the above described problems can be obviated or mitigated.

According to the present invention, there is provided a medical device for removing material from a body vessel, comprising a generally cylindrical housing having a leading inner edge, a helical member mounted in the housing so that a portion of the helical member is enclosed by the housing and a tip region of the helical member projects forwardly of the housing, means for rotating the helical member relative to the housing so that, in use, material is cut as it enters the housing by co-operation between the outer periphery of the helical member and the leading inner edge of the housing, and means in the housing for receiving cut material, characterized in that the helical member comprises a screw whose tip region is tapered for entering material to be removed from the body vessel.

With the above-described device, the tapered tip region of the screw assists in preventing accidental penetration of the wall of the body vessel during use or when being manoeuvd into position for use. It is also preferred for the leading outer edge of the housing to be radiussed. The instrument can be used percutaneously as a non-operative device.

It is preferred for the tip region of the screw to be tapered to a relatively blunt eg bullet shape which is not so sharp that it can penetrate the vessel wall, yet not so blunt that upon rotation it cannot enter material to be cleared from the body vessel. The tapered tip region of the screw can penetrate an obstruction, preferably by means of a "self-tapping" action and draw the housing along the body vessel so that the main cutting action occurs as a result of a scissor-like cutting co-operation between the crest of the thread of the screw and the inner leading edge of the housing. The crest of the screw thread is preferably relatively sharp. The screw thread may be a single or a multi start screw thread.

The outer surface of the screw cooperates with the inner leading edge of the housing to produce a circular cut. Such screw also serves, upon rotation, to transport material from the obstruction into the housing of the instrument. Conveniently, a chamber is provided in the housing to receive such material which can then be completely removed from the body vessel upon removal of the housing of the instrument therefrom. Thus, the material which has been cleared away is completely removed from the body vessel.

Alternatively, passages may be provided in the housing to enable the material which is in the housing to be continuously or periodically removed, eg by flushing from the vessel through a drain tube whilst the device is being used.

The means for rotating the screw is preferably an elongate flexible drive means which, more preferably, takes the form of a cable or the like mounted for rotary movement within a relatively fixed, flexible sleeve. The flexible sleeve preferably takes the form of a hollow helical spring which is preferably provided with a continuous cover or sheath to provide a smooth external surface for ease of passage along the vessel to be cleared.

The housing and the screw may be provided with a passage extending axially therethrough so that the they can be mounted on an elongate guide wire or the like. In use, it will be appreciated that such guide wire can be passed along the body vessel relatively easily because of its small diameter, and then the housing can be passed along the vessel whilst being guided by the wire so as to facilitate safe passage along the vessel to the site of the obstruction. The device will normally be used whilst the patient is conscious, and its progress along the vessel can be monitored by x-ray inspection.

In the case of devices according to the present invention designed for clearing arterial obstructions, the external diameter of the housing will be in the region of 2 to 4 mm. The device can be used for bioducts generally.

Embodiments of the present invention will now be described, by way of example, with reference to the accompanying drawings, in which:-

Fig. 1 is an axial section through a first example of surgical instrument according to the present invention;

Figs. 2 and 3 are schematic axial sections through parts of second and third examples of surgical instruments according to the present invention;

Fig. 4 is a schematic view illustrating a detail of the instrument of Fig. 3; and

Fig. 5 is an axial section through part of a fourth example of surgical instrument according to the present invention.

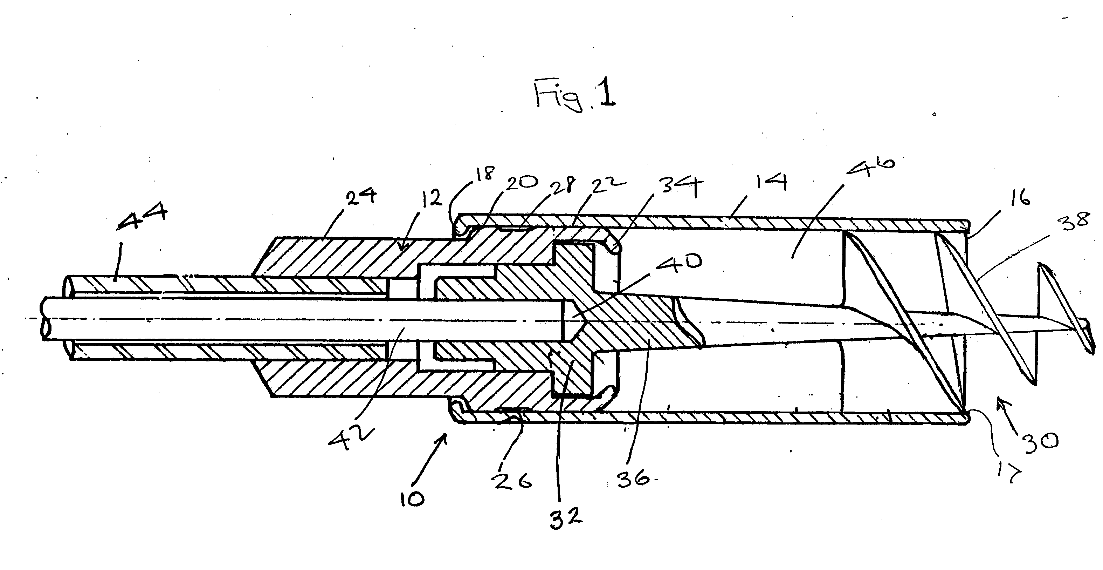

Referring now to Fig. 1, the surgical instrument illustrated therein is for clearing obstructions in coronary arteries. The instrument comprises a generally cylindrical housing 10 formed in two parts consisting of a hollow bush 12 and sleeve 14 extending forwardly of the bush 12. The sleeve 14 has an open leading or forward end 16 with a leading inner edge 17, and a rear end which has been inwardly deformed so as to define a stop 18. The inner edge 17 of the leading end 16 is sharp whilst the outer edge of the leading end 16 is radiussed to prevent artery wall damage. The stop 18 abuts against a step 20 provided on the external surface of the bush 12 between a larger diameter forward end portion 22 and a smaller diameter rear end portion 24 thereof. The sleeve 14 is slidable rearwardly (i.e. to the left as viewed in the drawing) relative to the bush 12 from an extended position in which (a) the stop 18 abuts against step 20 and (b) a pip 26 formed by localised inward deformation of the sleeve 14 engages in an annular groove 28 formed in the surface of the larger diameter forward end portion 22 of the bush 12. The engagement of the pip 26 in the groove 28 serves as a releasable detent mechanism for retaining the sleeve 14 in its extended condition as illustrated in the drawing.

The surgical instrument further includes a cutter member 30 in the form of a single start screw which is mounted in the housing 10 via a stepped root portion 32. The root portion 32 engages in a stepped bore of the bush 12 and is retained therein by an inwardly upset annular lip 34. The root portion 32 is rotatable relative to the bush 12 which acts as a bearing therefor. The root portion 32 carries a tapered shaft 36 which projects forwardly and axially of the sleeve 14 so as to pass through the open end 16 of the sleeve 14. At a position which is spaced forwardly of the bush 12, the shaft 36 is provided with integral screw threading 38 thereon. The screw threading 38 starts within the sleeve 14 and extends forwardly to the tip of the shaft 36, the screw threading tapering inwardly over a tip region thereof which is disposed externally of the sleeve 14. The forward end of the screw threading 38 and the forward end of the shaft 36 are relatively blunt and have no sharp edges whereby to minimise risk of inadvertent penetration of the arterial wall during use. The portion of the screw threading 38 which is disposed within the sleeve 14 has a peripheral surface (or crest) which is relatively sharp and which is in close sliding contact with the internal wall surface of the sleeve 14.

The root portion 32 has a blind axial bore 40 therein in which one end of a flexible drive cable 42 is firmly secured. The drive cable 42 extends through a flexible outer sleeve or sheath 44 and is rotatable relative thereto. In the drawing, only a short length of the cable 42 and sheath 44 are illustrated. In practice, these latter two parts will extend for a considerable distance rearwardly of the housing 10, with the cable 42 terminating externally of the sheath 44 in a formation which facilitates manual rotation of the cable 42 at a remote location.

In use, the housing 10 including the cutter member 30 is passed along an artery of a conscious patient until it meets the obstruction to be cleared. This is effected by appropriately manoeuvring the body 10 using the sheath 44 with the aid of an x-ray camera. When the housing 10 has been manoeuvred into the correct position along the artery, the cable 42 is rotated relative to the sheath 44 so as to cause the cutter member 30 to rotate in a direction in which it causes the forward end of the screw threading 38 to dig into the material of the obstruction and thereby draw itself and the housing 10 along the artery and further into the obstruction. A circular cutting action is provided by mutual cooperation of (a) the outer, relatively sharp, cutting edge of that portion of the screw threading 36 which is disposed within the sleeve 14 and (b) the leading inner edge 17 of the sleeve 14. The cut material is carried between the flights of the screw threading 38 within the sleeve 14 to be discharged into collection chamber 46 disposed within the sleeve 14 and between the screw threading 38 and the bush 12. When it is seen on the X-ray camera that the obstruction has been cleared, rotation of the cable 42 is stopped and the housing 10 is withdrawn by employing a pulling action on the sheath 44. Once the housing 10 has been removed from the artery 10, material which has been collected in the chamber 46 can be easily removed upon retraction of the sleeve 14 relative to the bush 12.

It will be appreciated that, because the main cutting action takes place by cooperation of the screw threading 38 with the sleeve 14, there is a minimum of risk that the wall of the artery will be inadvertently penetrated. In order to assist insertion of the housing 10 and to minimise risk of damage to the artery wall, the cutter edge of the leading end 16 of the sleeve 14 is conveniently radiussed. In order to facilitate withdrawal of the body 10 along the artery, the rear end of the smaller diameter portion 24 of the bush 12 and the rear edge of the sleeve 14 adjacent the stop 18 are both appropriately tapered.

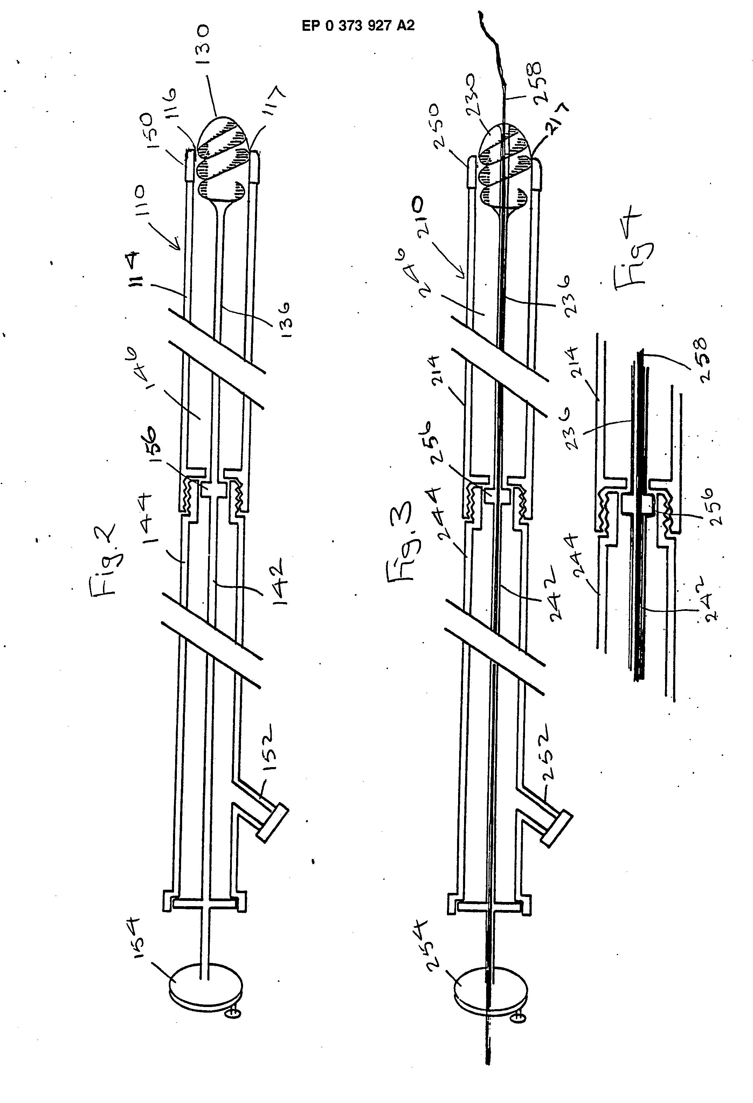

Referring now to Fig. 2, the surgical instrument illustrated therein is similar to that of Fig. 1 and similar parts are accorded the same reference numerals but in the 100 series. In this example, cutter member 130 takes the form of a screw which is rounded to a blunt bullet shape in its tip region to minimise risk of arterial wall penetration in use.

Sleeve 114 of housing 110 is flexible, but is fitted with a metal ring 150 to define open leading end 116. The rotary cutting action takes place between cutter member 130 and metal ring 150. The generally cylindrical housing 110 is internally screw-threaded at its rearward end to engage with external screw-threading on the forward end of sheath 144. The screw-threading is directed so that the screw-threaded joint between the housing 110 and the sheath 144 tends to be tightened during forward rotation of the cutter member 130 in use. The sheath 144 may have a smaller diameter than the housing 110. At its rear end, the sheath 144 is provided with a formation 152 to facilitate gripping of the instrument during use. Cable 142 passes out of the rear end of the sheath 144 and is connected to a hand wheel 154 (only schematically shown). The cable 142 is connected to shaft 136 by connector 156 which also acts as a bearing.

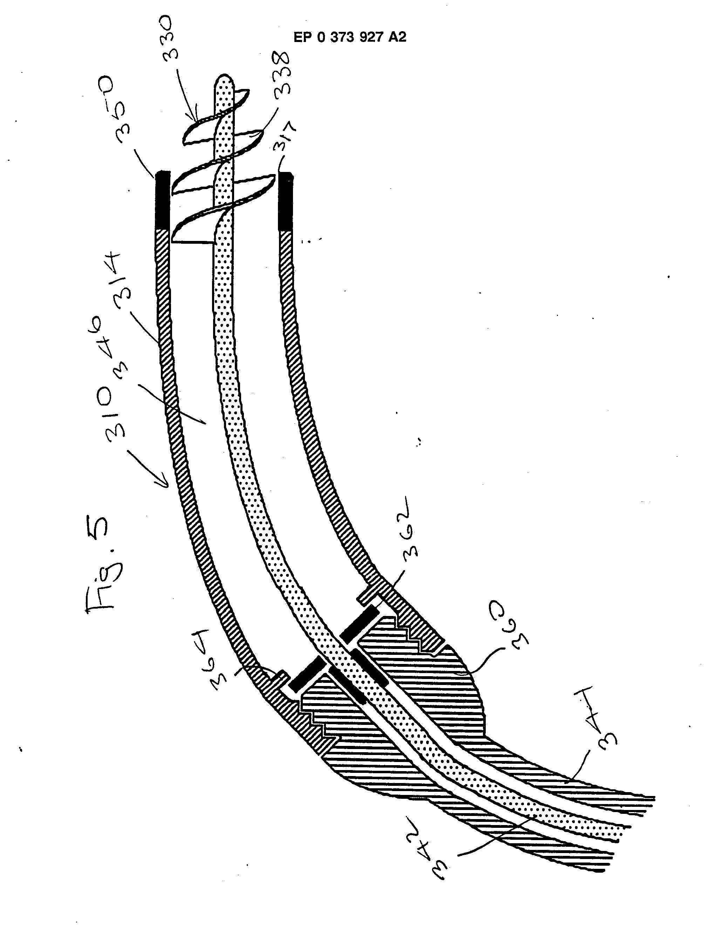

Referring now to Figs. 3 and 4, the surgical instrument illustrated therein is similar to that of Fig. 2 and similar parts are accorded the same reference numerals but in the 200 series. In this example, cutter member 230 and shaft 236 have aligned axial passages therethrough, and the cable 142 is replaced by a flexible tube 242 which is joined to the shaft 236 at connector 256 which is hollow. These axial passages typically have a diameter of about 0.4 - 0.46 mm. In use, a guide wire 258 typically having a diameter of about 0.36 mm is passed into and along the artery to a position beyond the location at which the obstruction exists. The generally cylindrical housing 210 can then be passed along the artery to the location of the obstruction whilst being continually guided by the wire 258 so as to minimise the risk of penetration of or damage to the wall of the artery.

Referring now to Fig. 5 of the drawings, the surgical instrument illustrated therein is similar to the preceding examples and similar parts are accorded the same reference numerals but in the 300 series. In this example, sheath 344 has a diameter which is substantially less than that of housing 310 except for an enlarged forward end portion 360 by which it is screw-engaged with body 310. A bush 356 around cable 342 assists in correctly locating the latter within the portion 360. A washer 362 in the housing 310 and lodged between the portion 360 and an internal rib 364 serves to limit passage of debris into the sheath 344 from chamber 346 and can be readily removed upon detachment of the housing 310 from the portion 360 to facilitate removal of debris from the chamber 346 after use.

## Claims

1. A medical device for removing material from a body vessel, comprising a generally cylindrical housing (10, 110, 210, 310) having a leading inner edge (17, 1176, 217, 317), a helical member (30, 130, 230, 330) mounted in the housing so that a portion of the helical member is enclosed by the housing and a tip region of the helical member projects forwardly of the housing, means (42, 142, 242, 342) for rotating the helical member relative to the housing so that, in use, material is cut as it enters the housing by co-operation between the outer periphery of the helical member and the leading inner edge of the housing, and means (46, 146, 246, 346) in the housing for receiving cut material, characterized in that the helical member (30, 130, 230, 330) comprises a screw whose tip region is tapered for entering material to be removed from the body vessel.

2. A device as claimed in claim 1, wherein the means for rotating the helical member is an elongate flexible drive means (42, 142, 242, 342).

3. A device as claimed in claim 1 or 2, wherein the leading outer edge of the housing is radiussed.

4. A device as claimed in any preceding claim, wherein there is a scissor-like cutting cooperation between the screw (30, 130, 230, 330) and the leading inner edge (17, 117, 217, 317).

5. A device as claimed in any preceding claim, wherein the housing includes an open ended sleeve (14, 150, 250, 350) which defines said leading inner edge (17, 117, 217, 317).

6. A device as claimed in any preceding claim wherein the tip of the screw (30, 130, 230, 330) is relatively blunt.

7. A device as claimed in any preceding claim, wherein the screw threading has a relatively sharp crest.

8. A device as claimed in any preceding claim, wherein the screw threading has a self-tapping action.

Fig.1

Fig.2

154  144  156  146  114  110  150  116  130  152  142  136  117

Fig.3

254  244  256  214  246  210  250  230  252  242  236  214  236  258  217

244  236  214  242  256  258

Fig.4

EP 0 373 927 A2

Fig. 5